# EUROPÄISCHE PATENTSCHRIFT

(11) **EP 0 965 115 B1**
(45) Veröffentlichungstag und Bekanntmachung des Hinweises auf die Patenterteilung: **20.03.2002**
(21) Anmeldenummer: 98908061.9
(22) Anmeldetag: 05.02.1998
(51) Int. Cl.: G08B 21/00, A61B 5/0404, A61B 5/00, H04M 1/72

(54) **MOBILTELEFON MIT GPS-EMPFÄNGER UND EKG-ELEKTRODEN**
MOBILE TELEPHONE WITH A GPS RECEIVER AND EKG ELECTRODES
TELEPHONE MOBILE AVEC RECEPTEUR GPS ET ELECTRODES ECG

(30) Priorität: 26.02.1997 DE 19707681
(43) Veröffentlichungstag der Anmeldung: 22.12.1999
(73) Patentinhaber: Erbel, Raimund, 45239 Essen (DE); Sack, Stefan, 45239 Essen (DE)
(72) Erfinder: Erbel, Raimund, 45239 Essen (DE); Sack, Stefan, 45239 Essen (DE)
(74) Vertreter: Patentanwälte Gesthuysen, von Rohr, Weidener, Häckel
(86) Internationale Anmeldenummer: EP9800626
(87) Internationale Veröffentlichungsnummer: WO9838611

(56) Entgegenhaltungen:
- EP-A- 0 310 379
- EP-A- 0 679 041
- DE-A- 19 639 492
- DE-U- 29 600 600
- GB-A- 2 285 135
- US-A- 5 544 661

## Beschreibung

Die Erfindung betrifft ein Mobiltelefon mit einem Gehäuse, einem Sender, mindestens einem Empfänger, einem Rufnummernspeicher und mit am Gehäuse angeordneten Tasten, wobei am Gehäuse mindestens eine, bei Betätigung die Wahl einer im Rufnummernspeicher gespeicherten Notrufnummer veranlassende in Größe und/oder Farbe hervorgehobene Notruftaste angeordnet ist.

Ein Mobiltelefon der eingangs genannten Art ist bereits aus der EP - A - 0 679 041 bekannt. Das bekannte Mobiltelefon weist eine Notruftaste auf, über die nach dem Betätigen selbsttägig über ein an sich bekanntes Mobilfunksystem eine Verbindung zu einer Zentralstelle hergestellt wird. In einem Speicher des Mobiltelefons gespeicherter Text wird nach dem Herstellen der Verbindung zur Zentralstelle übertragen. Die Zentralstelle ermittelt selbsttätig die Position des Mobiltelefons entweder durch Ortung mittels der Basisstationen des Mobilfunktsystems oder mittels von dem Mobiltelefon empfangener Positionsdaten, wenn das Mobiltelefon mit einer eigenen Ortungseinheit versehen ist.

Neben den bereits genannten Merkmalen weisen Mobiltelefone heute in der Regel ein LCD-Display und eine am Gehäuse angesetzte, häufig ausziehbare Antenne auf. Die wesentlichen Unterscheidungsmerkmale zwischen verschiedenen Mobiltelefonen sind zum einen Unterschiede im Design, Gewicht und der Funktionalität und andererseits die Zugehörigkeit zu einem der existierenden Mobilfunknetze. Neben dem analogen C-Netz existieren weiter die digitalen D1-, D2- und e-Netze. Weitere Mobilfunknetze sind darüber hinaus angedacht. Die existierenden Mobilfunknetze unterscheiden sich zum einen, wie bereits erwähnt, durch die ihnen zugrundeliegende Übermittlungstechnik, zum anderen durch den Grad der Flächendeckung, innerhalb derer über diese Mobilfunknetze telefoniert werden kann. Zielsetzung sämtlicher Netze ist es, daß die Teilnehmer von jedem beliebigen Punkt aus durch eine entsprechend ausgebaute Netzinfrastruktur Telefongespräche sowohl innerhalb des Mobilfunknetzes als auch unter Nutzung der leitungsgebundenen Telefonnetze führen können. Die existierenden Mobilfunknetze ermöglichen es zumindest teilweise, Telefonverbindungen auch aus abgelegenen Gegenden, z.B. während einer Wanderung, herzustellen.

Mit den bekannten Mobiltelefonen ist es selbstverständlich auch möglich, aus abgelegenen Gegenden, die sich innerhalb des Empfangs-/Sende-Bereiches des zugehörigen Mobilfunknetzes befinden, medizinische Notrufe abzusetzen. Hierzu kann beispielsweise die Notrufnummer in einem bei der überwiegenden Anzahl der Mobiltelefone vorhandenen Rufnummernspeicher abgelegt werden und über eine entsprechende Tastenkombination auf dem Mobiltelefon angerufen werden. Nun kann es jedoch zu Notfallsituationen kommen, in denen der Inhaber des Mobiltelefons selbst nicht mehr in der Lage ist, die entsprechende Tastenkombination einzugeben und auch nicht mehr in der Lage ist, einer eventuellen Begleitperson die entsprechende Tastenkombination seines speziellen Notrufes mitzuteilen. Dies spielt insbesondere dann eine Rolle, wenn der Inhaber des Mobiltelefons an einer lebensbedrohenden Erkrankung leidet und von einer speziellen medizinischen Institution betreut wird, der das Krankheitsbild ihres Patienten bekannt ist, und somit bei einem Notruf abgestimmte, geeignete Hilfsmaßnahmen einleiten kann. Solche abgestimmten Hilfsmaßnahmen sind über die landesweiten zentralen Notrufhummern in der Regel nicht zu gewährleisten.

Insbesondere bei Patienten mit Herz-Kreislauf-Erkrankungen treten die geschilderten Situationen statistisch gesehen regelmäßig auf. Erkrankungen des Herz-Kreislauf-Systems stehen an der Spitze des gesamten Krankheitsgeschehens in Deutschland. Die koronare Herzerkrankung ist in den westlichen Industrienationen die häufigste Todesursache. In Deutschland sind beispielsweise etwa 300.000 Menschen jährlich von einen Herzinfarkt betroffen. Bei einem solchen Herzinfarkt sind die häufigsten Komplikationen mit meist tödlichem Ausgang Rhythmusstörung und Herzinsuffizienz. Dabei erleiden in Deutschland 60.000 Menschen jährlich einen plötzlichen Herztod. Dieser Herztod ist gekennzeichnet durch einen plötzlichen Herzstillstand, der ohne sofortige Maßnahmen der kardiopulmonalen Reanimation oder Wiederbelebung regelmäßig tödlich endet. Ursachen für den plötzlichen Herztod sind zu 80-90% das sogenannte Kammerflattern oder Kammerflimmern (tachykarde Herzrhythmusstörungen), zu 10-15 % ein akuter Myokardinfarkt und zu 5 % bradykarde Rhythmusstörungen. Die Überlebensrate der Patienten, die einen plötzlichen Herztod erleiden, liegt zwischen 5 und 20 %. Für die Höhe der Überlebensrate ist ganz entscheidend maßgeblich, wie schnell geeignete Hilfsmaßnahmen ergriffen werden können.

Eine Vielzahl der Personen, die ein hohes Herzinfarktrisiko haben, sind sich nach entsprechenden Untersuchungen ihres Risikos bewußt und sind bestrebt, dieses Risiko zu minimieren, indem sie sich möglichst nicht in Situationen begeben, in denen die Ergreifung sofortiger Hilfsmaßnahmen nicht möglich ist So vermeiden es die betroffenen Personen beispielsweise, sich ohne Begleitpersonen an Orte zu begeben, an denen die Wahrscheinlichkeit im Falle eines Herzinfarktes sofort aufgefunden zu werden gering ist, da sie in diesen Fällen damit rechnen müssen, daß ihre Überlebenschancen beim Eintritt eines plötzlichen Herztodes nahezu gleich null sind.

Ausgehend vom Stand der Technik liegt der Erfindung die Aufgabe zugrunde, ein verbessertes und weiterentwickeltes Notrufmobilfunksystem zur Verfügung zu stellen.

Erfindungsgemäß ist die zuvor hergeleitete und aufgezeigte Aufgabe dadurch gelöst, daß mindestens zwei ― vorzugsweise vier ― mit dem Gehäuse verbundene, die Aufnahme von EKG-Signalen ermöglichende Elektroden vorgesehen sind und ein die EKG-Signale auswertender, sendefähig transformierender und an den Sender übermittelnder EKG-Signalwandler vorgesehen ist und daß die EKG-Elektroden auf der den Tasten abgewandten Rückseite des Gehäuses angeordnet sind.

Die erfindungsgemäße Ausgestaltung gewährleistet, daß in einem die körperlichen und geistigen Kräfte schwächenden medizinischen Notfall mit einem Druck auf die hervorgehobene Notruftaste entweder von dem Patienten selbst oder von einer Begleitperson, die nicht eingewiesen sein muß, ein Notruf an eine kompetente Überwachungszentrale abgesetzt werden kann. Sofern der Patient bei Bewußtsein ist, kann er dann seinen Standort und seine Beschwerden der Überwachungszentrale mitteilen. Über die bestehende Notrufleitung kann der Patient anschließend beruhigt und überwacht werden; ferner kann eine Anamnese erhoben und gezielte Hilfsmaßnahmen eingeleitet werden.

Im übrigen ermöglicht es die Erfindung, daß der Patient selbst oder eine Begleitperson die Elektroden auf die entkleidete Brust des Patienten legt, woraufhin ein EKG abgeleitet wird, das automatisch an die Überwachungszentrale übermittelt wird. Diese Ausgestaltung ermöglicht die Differenzialdiagnose eines akuten Herzinfarktes (ST-Streckenhebung) oder einer zugrundeliegenden Herzrhythmusstörung (Tachykardie, Bradykardie) bzw. eines elektrischen Herzstillstands (Asystolie). Die EKG-Erfassung und Übermittlung ermöglicht die Einschätzung der vitalen Bedrohung des Patienten und die Einleitung entsprechend geeigneter Notfallmaßnahmen. Auf Grundlage des übermittelten EKG ist auch eine Laien-Reanimation durch eine Begleitperson oder eine zufällig in der Nähe befindliche Person über das Mobiltelefon be-einflußbar und steuerbar.

Die zuletzt geschilderte Maßnahme ist dadurch besonders vorteilhaft weitergebildet, daß die EKG-Elektroden auf der den Tasten abgewandten Rückseite des Gehäuses angeordnet sind. In diesem Fall wird ein EGK einfach dadurch aufgenommen, daß das Mobiltelefon mit seiner Rückseite auf die entkleidete Brust des Patienten gelegt wird. Eine entsprechende Handhabung ist hierbei denkbar einfach.

Eine erste vorteilhafte Ausgestaltung erfährt die Erfindung dadurch, daß eine die Übermittlung von eine Identifikation ermöglichenden Daten bei der Betätigung der Notruftaste bewirkende Identifikationseinrichtung vorgesehen ist. Durch eine solche Identifikation ist gewährleistet, daß die Überwachungszentrale sofort über die beispielsweise in einem EDV-System gespeicherten Patientendaten auch für den Fall, daß der Patient bewußtlos ist, gezielte Notfallmaßnahmen einleiten kann. Mit der genannten Maßnahme ist beispielsweise auch eine Patientenidentifikation möglich, wenn der bewußtlose Patient von einer unbeteiligten Person aufgefunden und der Notruf von dieser Person abgesetzt worden ist. Eine solche Identifikation ist beispielsweise über die Rufnummer des Mobiltelefons möglich, die bei einigen Mobilfünknetzen bei der Herstellung einer Verbindung standardmäßig übertragen wird.

Ist weiter eine die Freigabe von Positionsdaten bei der Betätigung der Notruftaste bewirkende Freigabeeinrichtung vorgesehen, so ist gewährleistet, daß die Position des Patienten auch für den Fall festgestellt werden kann, in dem der Patient lediglich sozusagen mit letzter Kraft die Notruftaste betätigt hat. Eine derartige Ausgestaltung ermöglicht es auch in einer solchen Situation den Patienten aufzufinden und Soforthilfemaßnahmen einzuleiten. Einige Mobilfunknetze ermöglichen technisch eine solche Positionsbestimmung allein aufgrund der Tatsache, daß diese Mobilfunknetze in sogenannte Zellen aufgeteilt sind, und festgestellt werden kann, aus welcher Zelle des Mobilfunknetzes der Notruf getätigt wurde. In diesem Fall bedarf es keiner gesonderten Einrichtung zur Positionsbestimmung.

Eine sehr genaue Positionsbestimmung wird dadurch ermöglicht, daß ein externe Positionssignale empfangender und auswertender Positionsempfänger und ein die Signale des Positionsempfängers sendefähig transformierender und an den Sender übermittelnder Positionssignalwandler vorgesehen ist. Mit Hilfe eines solchen Positionsempfängers ist es beispielsweise möglich, aufgrund der Signale des Global-Positioning-Systems (GPS) die Position des Patienten auf wenige Meter genau zu bestimmen. Diese Signale werden dann über den Positionssignalwandler dem Sender zugeführt, der sie wiederum an die Überwachungszentrale übermittelt, so daß entsprechend in der Überwachungszentrale hochpräzise Angaben über die Position des Patienten vorliegen.

Schließlich erfährt das erfindungsgemäße Mobiltelefon eine weitere vorteilhafte Ausgestaltung dadurch, daß ein das Verlassen des Empfangs-/Sende-Bereiches des Mobilfunknetzes detektierender und die Ausgabe eines Warnsignals veranlassender Empfangsstärkensensor vorgesehen ist. Hierdurch ist gewährleistet, daß der Patient stets darüber informiert ist, ob im Augenblick tatsächlich eine Verbindung zur Überwachungszentrale im Falle eines Notrufes möglich ist.

Nachfolgend wird ein Ausführungsbeispiel der Erfindung anhand der Zeichnung beschrieben. Es zeigt
- Fig. 1: ein Ausfuhrungsbeispiel eines erfindungsgemäßen Mobiltelefons in einer Frontansicht und
- Fig.2: das Ausführungsbeispiel eines erfindungsgemäßen Mobiltelefons in einer Rückansicht.

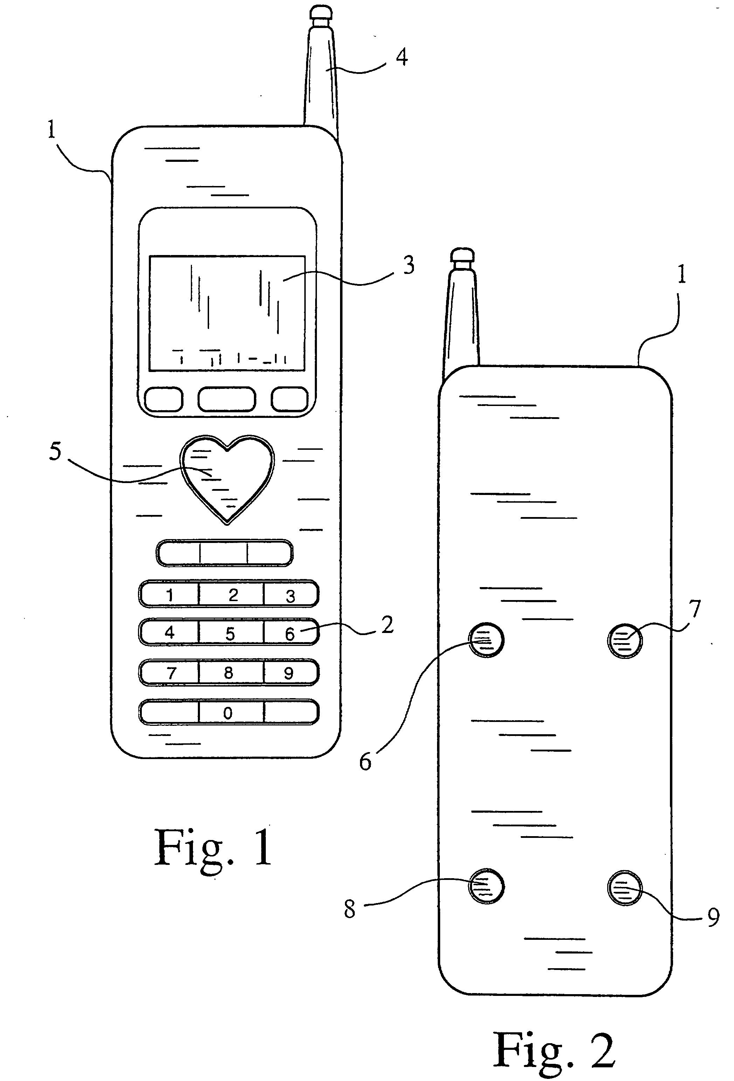

Das in Fig. 1 in einer Frontansicht dargestellte Ausführungsbeispiel eines erfindungsgemäßen Mobiltelefons weist auf ein Gehäuse 1, nicht dargestellt und im Gehäuse angeordnet einen Sender, mindestens einen Empfänger und einen Rufnummernspeicher und am Gehäuse 1 angeordnete Tasten 2. Darüber hinaus weist das Mobiltelefon ein LCD-Display 3 und eine Antenne 4 auf. Erfindungsgemäß ist am Gehäuse 1 eine, bei Betätigung die Wahl einer im Rufnummernspeicher gespeicherten Notrufnummer veranlassende, in Größe und Farbe hervorgehobene Notruftaste 5 angeordnet. In dem dargestellten Ausführungsbeispiel ist die Notruftaste 5 deutlich größer als die üblichen Tasten 2. Zur weiteren Kenntlichmachung ihrer Funktion ist die Notruftaste 5 herzförmig ausgestaltet und im Ausführungsbeispiel rot abgesetzt hervorgehoben.

Die gemäß verschiedenen Ausgestaltungen des erfindungsgemäßen Mobiltelefons vorgesehenen Identifikations- und Freigabe-Einrichtungen, Positionsempfänger, Positionssignalwandler, EKG-Signalwandler und Empfangsstärkensensor sind als elektronische Baueinheiten ausgeführt und, in der Zeichnung nicht dargestellt, im Gehäuse 1 angeordnet.

In Fig. 2 der Zeichnung ist schließlich das Ausführungsbeispiel eines erfindungsgemäßen Mobiltelefons in einer Rückansicht dargestellt. In der Rückansicht ist deutlich zu erkennen, daß auf der den Tasten abgewandten Rückseite des Gehäuses 1 vier EKG-Elektroden 6, 7, 8, 9 angeordnet sind, die es bei dem mit der Rückseite auf die entkleidete Brust eines Patienten gelegten Mobiltelefon ermöglichen, ein EKG abzuleiten.

Weitere denkbare Ausgestaltungen des erfindungsgemäßen Mobiltelefons sind beispielsweise eine zusätzliche Beschriftung der Notruftaste 5 etwa mit dem Wort "Notruf" und/oder die Anbringung von Befestigungsmöglichkeiten am Gehäuse 1, die über ein Umhängeband das Tragen des erfindungsgemäßen Mobiltelefons auf der Brust ermöglichen.

## Patentansprüche

1. Mobiltelefon mit einem Gehäuse (1), einem Sender, mindestens einem Empfänger, einem Rufnummernspeicher und mit am Gehäuse (1) angeordneten Tasten (2), wobei am Gehäuse (1) mindestens eine, bei Betätigung die Wahl einer im Rufnummernspeicher gespeicherten Notrufnummer veranlassende in Größe und/oder Farbe hervorgehobene Notruftaste (5) angeordnet ist, **dadurch gekennzeichnet, daß** mindestens zwei - vorzugsweise vier - mit dem Gehäuse (1) verbundene, die Aufnahme von EKG-Signalen ermöglichende Elektroden (6, 7, 8, 9) vorgesehen sind und ein die EKG-Signale auswertender, sendefähig transformierender und an den Sender übermittelnder EKG-Signalwandler vorgesehen ist und daß die EKG-Elektroden (6, 7, 8, 9) auf der den Tasten (2) abgewandten Rückseite des Gehäuses (1) angeordnet sind.

2. Mobiltelefon nach Anspruch 1, **dadurch gekennzeichnet, daß** eine die Übermittlung von eine Identifikation ermöglichenden Daten bei der Betätigung der Notruftaste (5) bewirkende Identifikationseinrichtung vorgesehen ist.

3. Mobiltelefon nach Anspruch 1 oder 2, **dadurch gekennzeichnet, daß** eine die Freigabe von Positionsdaten bei der Betätigung der Notruftaste (5) bewirkende Freigabeeinrichtung vorgesehen ist.

4. Mobiltelefon nach Anspruch 3, **dadurch gekennzeichnet, daß** ein externe Positionssignale empfangender und auswertender Positionsempfänger und ein die Signale des Positionsempfängers sendefähig transformierender und an den Sender übermittelnder Positionssignalwandler vorgesehen ist.

5. Mobiltelefon nach einem der Ansprüche 1 bis 4, **dadurch gekennzeichnet, daß** ein das Verlassen des Empfangs-/Sende-Bereiches des Mobilfunknetzes detektierender und die Ausgabe eines Warnsignals veranlassender Empfangsstärkensensor vorgesehen ist.

## Claims

1. Mobile telephone with a housing (1), a transmitter, at least one receiver, a call number memory and keys (2) located on the housing (1), wherein at least one emergency key, which is rendered prominent in size and/or color and which, when activated, causes dialing of an emergency number stored in the call number memory, is arranged on the housing (1) **characterized in that** at least two, preferably four electrodes (6, 7, 8, 9) are provided that enable reception of electrocardiogram (EKG) signals and are connected to the housing (1), and that an EKG signal converter is provided that evaluates the EKG signals, transforms them to make them suitable for transmission and transmits them to the transmitter and that the EKG electrodes (6, 7, 8, 9) are arranged on the back of the housing (1), opposite the keys (2).

2. Mobile telephone according to claim 1, **characterized in that** there is an identification means which causes transmission of data which enable identification when the emergency key (5) is activated.

3. Mobile telephone according to claim 1 or 2, **characterized in that** there is a release means which causes release of position data when the emergency key (5) is activated.

4. Mobile telephone according to claim 3, **characterized in that** there is a position receiver which receives and evaluates external position signals and a position signal converter which transforms the signals of the position receiver to make them suitable for transmission and transmits them to the transmitter.

5. Mobile telephone according to any one of claims 1 to 4, **characterized in that** there is a receiving level sensor which causes output of a warning signal and which detects departure form the receiving/transmitting area of the mobile radiotelephone network.

## Revendications

1. Téléphone mobile comportant un boîtier (1), un émetteur, au moins un récepteur, une mémoire de numéros d'appel et des touches (2) disposées sur le boîtier (1), au moins une touche d'appel d'urgence (5) mise en valeur par la taille et/ou la couleur et ordonnant la sélection d'un numéro d'appel d'urgence mémorisé dans la mémoire des numéros d'appel, **caractérisé en ce qu'**au moins deux, de préférence quatre, électrodes (6, 7, 8, 9) reliées au boîtier (1) et permettant l'enregistrement de signaux d'électrocardiogramme et un convertisseur de signal d'électrocardiogramme analysant les signaux d'électrocardiogramme, les transformant de façon à pouvoir les envoyer et les transmettant à l'émetteur et **en ce que** les électrodes d'électrocardiogramme (6, 7, 8, 9) sont disposées sur la face arrière, opposée aux touches (2) du boîtier (1).

2. Téléphone mobile selon la revendication 1, **caractérisé en ce qu'**il est prévu un système d'identification entraînant la transmission de données permettant une identification lors de l'actionnement de la touche d'appel d'urgence (5).

3. Téléphone mobile selon la revendication 1 ou 2, **caractérisé en ce qu'**il est prévu un système de validation entraînant la validation de données de position lors de l'actionnement de la touche d'appel d'urgence (5).

4. Téléphone mobile selon la revendication 3, **caractérisé en ce qu'**il est prévu un récepteur de position recevant et analysant des signaux de position externes et un convertisseur de signal de position transformant les signaux du récepteur de position pour pouvoir les envoyer et les transmettant à l'émetteur.

5. Téléphone mobile selon l'une quelconque des revendications 1 à 4, **caractérisé en ce qu'**il est prévu un capteur d'intensité de réception qui détecte l'abandon de la plage de réception / d'émission du réseau de téléphonie mobile et ordonnant l'émission d'un signal d'avertissement.
